Europäisches Patentamt

European Patent Office

Office européen des brevets

(19)

(11) Publication number: **0 237 189**
**A2**

(12) # EUROPEAN PATENT APPLICATION

(21) Application number: **87301150.6**

(22) Date of filing: **10.02.87**

(51) Int. Cl.⁴: **A61K 37/02**

(30) Priority: **14.03.86 US 839857**
**20.10.86 US 921287**
**10.02.86 US 828112**

(43) Date of publication of application:
**16.09.87 Bulletin 87/38**

(84) Designated Contracting States:
**BE CH DE ES FR GB GR IT LI NL SE**

(71) Applicant: **REPLIGEN CORPORATION**
**101 Binney Street**
**Cambridge Massachusetts 02142(US)**

(72) Inventor: **Pigiet, Vincent P.**
**666 Main Street, Apt. L-1**
**Winchester Massachusetts 01898(US)**
Inventor: **Millis, Cynthia D.**
**3720 West 8th Avenue, Apt. 5**
**Vancouver British Columbia, V6R 1Z3(CA)**

(74) Representative: **Perry, Robert Edward et al**
**GILL JENNINGS & EVERY 53-64 Chancery**
**Lane**
**London WC2A 1HN(GB)**

(54) Therapeutic and related uses of dithiol peptides.

(57) Metal-catalysed oxidative damage can be prevented using thioredoxin or thioredoxin-derived, or thioredoxin-like, dithiol peptide.

EP 0 237 189 A2

## THERAPEUTIC AND RELATED USES OF DITHIOL PEPTIDES

This invention relates to therapeutic and related uses of dithiol peptides. Another therapeutic use of such compounds is described and claimed in the other European Patent Application filed on 10th February 1987 which claims priority from US Patent Application No. 828,112 filed on 10th February 1986 (Agents Ref: 70/2797/02). Some of the description herein duplicates the text therein, and Example 1 herein is also in EP-A-0208539 (as Example 4 therein).

Oxygen-free radicals (oxy-radicals) are partially reduced forms of oxygen. Transition metal catalysis appears to have an integral role in the generation of oxy-radicals that initiate oxidative damage. The participation of oxy-radicals has been implicated in several biological reactions and disease states which include inflammation, post-ischemic tissue injury, ageing, carcinogenesis (i.e. DNA damage), drug action and drug toxicity, lipid peroxidation and protein degradation. For reviews, see Oxygen and Living Processes: An Interdisciplinary Approach, ed. Gilbert (1981), Springer Verlag, New York; Halliwell, B. and Gutteridge, J. M. C. (1984) Biochem. J. 219:1-14; Aust, S.D., Moorhouse, L.A. and Thomas C.E. (1985) J. Free Radical Biol. and Med. 1:3-25; and Oxidative Stress, ed. Siss (1985) Academic Press, New York and London.

The chemistry of oxygen and partially-reduced forms of oxygen (i.e. superoxide $O_2^{\cdot-}/HO^{\cdot}$, hydrogen peroxide $H_2O_2$, and hydroxyl radical $^{\cdot}OH$) with transition metals have been studied principally with iron and copper. The generation of superoxide either chemically or enzymatically is considered to participate in the metal-catalyzed Haber-Weiss reaction (or the $O_2^{\cdot-}$-driven Haber-Weiss):

$$Fe^{3+} + O_2^{\cdot-} \text{------} Fe^{2+} + O_2$$
$$O_2^{\cdot-} + HO_2 \text{------} H_2O_2 + O_2$$
$$Fe^{2+} + H_2O_2 \text{------} Fe^{3+} + OH + \bullet OH$$

These reactions are considered to be non-reactions in the absence of transition metals (i.e., rates of reaction of uncatalyzed reactions are slow). Certain iron-chelators such as ethylenediaminetetraacetic acid (EDTA) promote the iron-catalyzed Haber-Weiss reaction presumably by chelating the $Fe^{3+}$-(ferric form) and maintaining it in a suitable form. On the other hand, other chelators such as desferrioxamine (Desferal ® mesylate, Ciba-Geigy, Hawthorne, NY) inhibit the iron-catalyzed Haber-Weiss reaction presumably by inhibiting either the reduction of $Fe^{3+}$- or the $Fe^{2+}$-catalyzed decomposition of $H_2O_2$. Several thiol compounds and biological reductants (i.e., gluthathione [GSH], ascorbate, dithiothreitol [DTT] have been shown in various systems to either promote the iron-catalyzed Haber-Weiss reaction through reduction of the iron or inhibit free-radical reactions at concentrations much higher than that of the metal.

### Brief Summary of the Invention

The novel process of the subject invention comprises the use of thioredoxin compounds to prevent metal catalyzed oxidative damage in, for example, biological reactions and disease states. Since oxidative damaging reactions (i.e., Haber-Weiss) require metal-catalysis for initiation and are considered to be non-reaction in the absence of metals, oxidative damage of any sort may be inhibited by thioredoxin via metal chelation of contaminating metals or by anti-oxidative properties inherent to sulfhydryls or other amino acids. (Metals are ubiquitous in water and biological systems and therefore, unless care has been taken to remove them, will be present.) More specifically, the present invention concerns the use of thioredoxin compounds to inhibit metal-catalyzed peroxidation of lipids in solution or in membranes in vitro at a concentration equal to that of the metal. Further, thioredoxin can be used to interact with iron and copper in solution. Thus, this enzyme and polypeptides with similar sequences, referred to herein as "thioredoxin compounds," can be employed for in vitro or in vivo use to prevent metal-catalyzed oxidative damage or stress. Specific examples include use in metal-chelation therapy for ischemia, as an inhibitor of lipid peroxidation due to drug action or toxicity, as an anti-inflammatory agent, and in prevention of DNA damage. Additionally and advantageously, the thioredoxin compounds disclosed herein can be used to maintain the biological activity of proteins or other biologically-active compounds, for example, antibiotics and proteins, which would otherwise be inactivated by oxidation in the presence of contaminating or added transition metals. Thioredoxin can be used as a general anti-oxidant in the food and cosmetic industry since it inhibits peroxidation of lipids, a constituent of both food and cosmetics.

### Detailed Disclosure of the Invention

The thioredoxin compounds can be used in in vitro systems to prevent inactivation of biologically-active compounds by oxidation in the presence of contaminating or added transition metals, e.g., iron, copper, manganese, vanadium, and the like. The level of thioredoxin compound which can be used in such in vitro systems can range from about 1 $\mu$M to about 100 mM. The optimum level of usage can be readily determined by a person skilled in the art. This optimum level will be affected by the level of metal contamination present. Higher metal levels will require higher levels of thioredoxin compound to maintain the biological activity of the biologically-active compound. Thus, it is readily evident that aliquots of any given reaction vessel can be treated with titered amounts of thioredoxin compound until a stabilization of biological activity is assayed. This effective amount of thioredoxin compound to stabilize biological activity then can be scaled-up to stabilize the biologically-active compound in the reaction vessel. Where production of a biologically-active compound, for example an antibiotic or protein or polypeptide, is done on a continuous basis rather than in a batch system, then effective amounts of thioredoxin compound to stabilize the biological activity of the desired product can be fed into the product stream by well-known means. The quantity of thioredoxin compound to feed will again be determined upon a representative sample, as described above.

The use of the thioredoxin compounds to treat in vivo various disease states, as described above, can be done by administering the compounds, generally systematically, to those humans or animals suffering from the disease. The particular mode of administration will be dependent upon the nature and location of the particular disease state. For example, treatment of an inflammation condition would, advantageously, be done as close to the inflamed site as possible. Where the inflammation is an inflamed joint, then injection at the site would generally be the most effective. However, treatment can also include preparations administered nasally, orally, rectally, or applied topically. In general, the mode of administration will follow known means for administering medicinals to treat similar disease states. The level of thioredoxin compound which can be used will range from about 1 $\mu$g/kg to about 100 mg/kg.

Fluid unit dosage forms for oral administration such as syrups, elixirs, and suspensions can be prepared wherein each teaspoonful of composition contains a predetermined amount of thioredoxin compound for administration. The thioredoxin compound can be dissolved in an aqueous vehicle together with sugar, flavoring agents and preservatives to form a syrup. An elixir is prepared by using a hydroalcoholic vehicle with suitable sweetener together with a flavoring agent.

For parenteral administration, fluid unit dosage forms are prepared utilizing the thioredoxin compound and a sterile vehicle, water being preferred.

The thioredoxin compound, depending on the form and concentration used, can be dissolved in the vehicle. In preparing solutions, the thioredoxin compound can be dissolved in water for injection, and filter sterilized before filling into a suitable vial or ampule and sealing. Advantageously, adjuvants such as a local anesthetic, preservative and buffering agents can be dissolved in the vehicle. Thioredoxin may be advantageously administered in the reduced form. The reduced form can be obtained by treating the oxidized form with any number of conventional thiol reductants, such as dithiothreitol.

In addition to oral and parenteral administration, the rectal and vaginal routes can be utilized. The thioredoxin compound can be administered by means of a suppository. A vehicle which has a melting point at about body temperature or one that is readily soluble can be utilized. For example, cocoa butter and various polyethylene glycols - (Carbowaxes) can serve as the vehicle.

For intranasal instillation, fluid unit dosage forms are prepared utilizing the thioredoxin compound and a suitable pharmaceutical vehicle, water being preferred, or by dry powder for insufflation.

For use as aerosols, the thioredoxin compound can be packaged in a pressurized aerosol container together with a gaseous or liquefied propellant, for example, dichlorodifluoromethane, carbon dioxide, nitrogen, propane, and the like, with the usual adjuvants such as cosolvents and wetting agents, as may be necessary or desirable.

The term "unit dosage form" as used herein refers to physically discrete units suitable as unitary dosages for human and animal subjects, each unit containing a predetermined quantity of thioredoxin compound calculated to produce the desired therapeutic effect in association with the required pharmaceutical diluent, carrier or vehicle. The specifications for the novel unit dosage forms of this invention are dictated by and are directly dependent on (a) the unique characteristics of the active material, i.e., thioredoxin compound, and the particular therapeutic effect to be achieved, and - (b) the limitation inherent in the art of compounding such an active material for therapeutic use in humans. Examples of suitable unit dosage forms in accord with this invention are tablets, capsules, troches, suppositories, powder packets, wafers, cachets, teaspoonfuls, tablespoonfuls, dropperfuls, ampuls, vials, segregated multiples of any of the foregoing, and other forms as herein described. For example, the thioredoxin compound to be employed as an anti-inflammatory agent can be easily prepared in unit dosage form with the employment of pharmaceutical materials which themselves are available in the art and can be prepared by established procedures. The quantity of thioredoxin compound suitable for various dosage forms can be readily determined. Suitable dosage adjustments can be readily made to meet the severity of the treated condition while taking into account the age, weight, and overall condition of the host being treated.

Thioredoxins are low molecular weight dithiol proteins that have the ability to reduce disulfides in typical organic compounds such as Elman's reagent or disulfides as they exist naturally in a variety of proteins (Holmgren, A [1981] Trends in Biochemical Science, 6, 26-39).

Thioredoxin and thioredoxin-derived or thioredoxin-like, dithiol peptides within the scope of the subject invention, and referred to collectively as thioredoxin compounds, are exemplified by the following compounds:

(1) thioredoxin isolated from Escherichia coli (Laurent, T.C., Moore, /E.C., and Reichard, P. - [1964] J. Biol. Chem., 239, 3436-3445);

(2) thioredoxins isolated from other sources, e.g., thioredoxin isolated from yeast (Porque, G.P., Baldesten, A., and Reichard, P. [1970] J. Biol. Chem., 245, 2362-2379); Cyanobacterium - (Gleason, F.K.. and Holmgren, A. [1983] in "Thioredoxins, Structure and Function" [P. Gadal, ed.] Editions du Centre National de la Recherche Scientifique); rat (Guerara, J., Moore E.C., and Ward, D.NM. [1983] ibid; T₄ bacteriophage - (Soderberg, B-O, Sjoberg, B-M, Sonnerstam, U., and Branden, C-I [1978] Proc. Natl. Acad. Sci. USA, 75, 5827-5830); purification of mammalian thioredoxin (Luthman, M. and Holmgren, A. [1982] Biochem. 121, 6628-6633); further, thioredoxin from a human source can be used in the subject invention;

(3) thioredoxin-derived dithiol peptides representing peptides produced by cleavage of intact thioredoxins, as described in Example 1, infra. One such example of this class of thioredoxin-derived peptides is the fragment containing residues 1 through 37 (i.e., $T_{1-37}$) produced by cyanogen bromide cleavage of thioredoxin from E. coli. The important feature of these thioredoxin-derived, and thioredoxin-like, dithiol peptides is that they contain the redox-active peptide sequence, Cys-X-Y-Cys, wherein X and Y can be any of the 20 amino acids. For example, the redox-active peptide sequence from E. coli thioredoxin is Cys-Gly-Pro-Cys - (Cys = cysteine, Gly-glycine, Pro = proline). Also the redox-active sequences, Cys-Gly-Pro-Cys-Lys or Trp-Cys-Gly-Pro-Cys-Lys can be used (Lys-lysine): and

(4) thioredoxin-like dithiol peptides that inter alia have the intrinsic ability to catalyze the reduction of protein disulfides. These thioredoxin-like dithiol peptides will generally have the characteristic of containing a pair of cysteine residues which form a redox-active disulfide. This example includes peptides, derived from natural sources or constructed synthetically, that include the same redox-active peptide sequence disclosed above, for example, as in E. coli thioredoxin, Cys-Gly-Pro-Cys, Cys-Gly-Pro-Cys-Lys, or Trp-Cys-Gly-Pro-Cys-Lys, or analogous sequences from other thioredoxins such as that encoded for by T4 bacteriophage, Cys-Val-Tyr-Cys (Cys = cysteine, Val = valine, Tyr = tyrosine) (Soderberg, B-O, Sjoberg, B-M, Sonnerstam, U., and Branden, C-I - [1978] Proc. Natl. Acad. Sci. USA, 75, 5827-5830). Other thierodoxin-like peptides include the class of seed proteins called purothionins that have intrinsic thioredoxin-like activity (Wada, K. and Buchanan, B.B. [1983] in "Thioredoxins, Structure and Function" [Gadal, P., ed], Editions du Centre National de la Recherche Scientifique).

Thioredoxin is purified from a commercial source of E. coil, strain B (Grain Processing Corp., Minneapolis, MN) or from any of a number of common strains of E. coli grown by standard procedures (Pigiet, V. and Conley, R.R. [1977] J. Biol. Chem., 252, 6367-6372). The protein is purified using standard procedures including chromatography on ion exchange and molecular seive columns (Williams, C.H., Zanetti, G., Arscott, L.D. and McAllister, J.K. [1967] J. Biol. Chem. 242, 5226-5231; McEvoy, M., Lantz, C., Lunn, C.A. and Pigiet, V. [1981] J. Biol. Chem. 256, 6646-6650).

Thioredoxin protein is assayed immunologically using quantitative rocket immunoelectrophoresis as described in McEvoy et al., supra.

Following are examples which illustrate the process of the invention, including the best mode. These examples should not be construed as limiting. All solvent mixture proportions are by volume unless otherwise noted.

Example 1--Thioredoxin Fragments $T_{1-37}$ and $T_{19-36}$.

(a) Production of $T_{1-37}$ by Cyanogen Bromide Cleavage

A sample of E. coli thioredoxin was dialyzed in water for 12 hr at 4°C. Five ml was dried and resuspended in 70% formic acid. Cyanogen bromide (Sigma Chemical) was dissolved in 70% formic acid and added to thioredoxin in a 50-fold molar excess of methionine. The solution was purged with nitrogen and incubated at room temperature in the dark for 24 hr. At the completion of the cleavage reaction the solution was dried under nitrogen, resuspended in sodium acetate buffer and adjusted to pH 8.5 with ammonium hydroxide.

Samples were loaded onto a Waters $\mu$-Bondapack C-18 column (Trademark of Waters Associates, Inc., Milford, MA) attached to a Beckman Model 421 system (Trademark of Beckman Instruments, Inc., Fullerton, CA) monitored at 214 nm. The solvent system employed was 0.1% trifluoracetic acid (Buffer A) and 0.08% trifluoroacetic acid in acetonitrile (Buffer B). A gradient from 0% to 60% B over 30 min was used to separate the peptides at a flow rate of 2 ml/min.

Thioredoxin was cleaved by CNBr into two fragments, $T_{1-37}$ and $T_{38-108}$, eluting at 44% and 51% buffer B, respectively. Amino acid analysis identified and confirmed the composition of both peptides. (Holmgren, A. and Reichard, P. [1967] Eur. J. Biochem. 2, 187-196). $T_{1-37}$ contained the active site of the enzyme. The two peptides recovered accounted for 69% of the starting material. Unreacted thioredoxin accounted for 12-15% of the loss, while HPLC separation may be responsible for the additional losses.

#### (b) Production of $T_{19-36}$ by Trypsin Cleavage

After HPLC separation, described above, $T_{1-37}$ was pooled, dried, and resuspended in sodium acetate buffer and adjusted to pH 8.0 with NH₄OH. An aliquot of trypsin (Sigma Chemical) was added to the incubation at 1% (w/w) of peptide concentration. The reaction mixture was incubated at 37° for 1 hr. Separation of trypsin fragments was done by HPLC as for the cyanogen bromide fragments.

Trypsin digestion of the $T_{1-37}$ peptide yielded two peptides, $T_{4-18}$ and $T_{19-36}$, which were resolved by HPLC, eluting at 31% and 45% in buffer B, respectively. Amino acid analysis revealed that the species eluting at 31% B contained 15 amino acids and corresponds to the active site peptide. $T_{19-36}$. Incubation of 90 nmoles of $T_{1-37}$ produced 80 nmoles $T_{19-36}$ after separation by HPLC with a yield of 88%.

#### Example 2

Lipid peroxidation is a process which is known to occur both in plants and animals during certain toxic, drug-induced and disease states (Elstmer, E.F. [1982] Anr. Rev. Plant Phys. 33:73-96; Kappus, H. [1985] In: "Oxidative Stress" [H. Sies, Ed] 273-310, Academic Press, New York and London). Lipid peroxidation involves the formation and propagation of lipid radicals, uptake of oxygen, rearrangement of double bonds in unsaturated lipid and eventual destruction of membrane lipids producing a variety of breakdown products, one of which is malondialdehyde (Kappus, H. [1985] supra).

Peroxidation of microsomal lipid as assessed by malondialdehyde (MDA) formation was inhibited by thioredoxin (oxidized and reduced). All solutions were chelexed prior to use. Incubations containing rat liver microsomes (0.5 mg microsomal protein ml), adenosine diphosphate (ADP)-$Fe^3$ (1.7 mM ADP, 0.1 mM FeCl₃) and nicotinamide adenine dinucleoside phosphate (NADPH) (0.1 mM) were inhibited by thioredoxin at a concentration equal to that of iron (i.e., 0.1 mM). Varying the concentration of thioredoxin produces a titration curve in a plot of MDA formation versus concentration of thioredoxin. The slope of the curve was relatively steep, suggesting a very strong interaction with the iron. The ability of both oxidized and reduced thioredoxin to act similarly may reflect the ability of microsomes to reduced thioredoxin and thioredoxin's ability to associate with the lipid bilayer of the microsomes. DTT (0.4 and 1.28 mM) promoted the peroxidation of microsomal lipid when included in the incubation mixtures as assessed by increased amounts of MDA production (1,016% and 781% of control, respectively). This shows that thioredoxin's action is unique in that other dithiols cannot mimic it.

#### Example 3

Thioredoxin interacts with transition metals as assessed by HPLC. The presence of iron resulted in the disappearance of the peak corresponding to native thioredoxin and a concomitant appearance of a second species. Formation of this second species was dependent upon the concentration of iron. Thus thioredoxin interacts with iron to form a stable complex. Results show that thioredoxin chelates iron with an affinity comparable to or better than that of EDTA. Similar results were also observed with copper.

#### Example 4

Upon substituting the thioredoxin in Examples 2 and 3 with the thioredoxin fragments $T_{1-37}$ or $T_{19-36}$ obtained in Example 1, or $T_{31-36}$ (Example 8) there are obtained essentially the same results.

#### Example 5

Upon substituting the thioredoxin in Examples 2 and 3 with a thioredoxin-derived, or thioredoxin-like, dithiol peptide comprising the redox-active peptide sequence Cys-X-Y-Cys-Lys, Cys-X-Y-Cys, or Trp-Cys-X-Y-Cys-Lys-, wherein X and Y can be the same or different and are any of the 20 amino acids, there are obtained essentially the same results.

Example 6

Thioredoxin was compared to several iron chelators (i.e., EDTA and desferrioxamine) in its ability to prevent radical formation. The iron caralyzed formation of the hydroxyl radical - (generated as $O_2^{-}$ by hypoxanthine, and xanthine oxidase) was monitored by reaction with dimethyl sulfoxide which yields formaldehyde (Graf, E. et al. [1984] J. Biol. Chem. 259: 3620-3624). Triplicate 1.0 ml samples of 50 mM Tris pH 7.4, 50 $\mu$M $Fe^{3+}$, 250 $\mu$M chelator (or thioredoxin), 50 mM dimethyl sulfoxide, 300 $\mu$M hypoxanthine, and 18 Milli units of xanthine oxidase were incubated at 37°C for 30 min. All solutions were passed over a chelex column prior to use. Reactions were initiated by the addition of xanthine oxidase and terminated by the addition of 50 $\mu$l 100% trichloroacetic acid. Formaldehyde was determined colorimetrically by the Hantzsch reaction (Nash, T. [1953] Biochem. J. 55: 416-421). Thioredoxin (both oxidized and reduced) and desferrioxamine at 250 $\mu$M inhibited all catalytic activity of the iron in that no formaldehyde was detected, whereas EDTA at 250 $\mu$M gave a rate of 7.1 ± 0.8 nmols formaldehyde/30 min as compared to control (no chelator) of 26.9 ± 1.3 nmols of formaldehyde/30 min. Thus thioredoxin behaves as desferrioxamine in inhibiting radical formation whereas EDTA cannot.

Example 7

Thioredoxin inhibited the peroxidation of arachidonic acid micelles in an iron-catalyzed superoxide-dependent lipid peroxidation system. All solutions were treated with chelex prior to use to remove extraneous metal ions. Incubations contained arachidonic acid (0.2 mg/ml in suspension with LUBROL-PX [Sigma Chemical Company, St. Louis, MO] 0.08% w/v), adenosine diphosphate - (ADP) - $Fe^{3+}$ (0.5 mM ADP, 0.1 mM $FeCl_3$), xanthine (0.33 mM) and xanthine oxidase (0.1 units.ml). MDA formation was completely inhibited by thioredoxin (oxidized or reduced) at a concentration equal to that of iron (i.e., 0.1 mM),. Inhibition of MDA formation was linearly proportional to the concentration of oxidized thioredoxin. A similar curve was generated using desferrioxamine, and at 0.1 mM MDA inhibition was 100%. A similar curve was generated using reduced thioredoxin; however, inhibition was 100% at approximately 0.05 mM thioredoxin. The ability of oxidized thioredoxin to cause a concentration-dependent decrease in MDA formation may be due to the fact that (1) the formation of superoxide or ferrous ion (via superoxide's reduction of ferric ion) may reduce oxidized thioredoxin and it may now chelate the iron, or (2) oxidized thioredoxin may act as a radical scavenger either through sulfhydryls or other amino acid residues, i.e., tryptophan, histidene, etc. Thioredoxin, which was reduced and the thiols carboxymethylated by iodoacetamide, caused a small concentration-dependent inhibition of MDA formation amounting to a 23% inhibition at 0.1 mM. This result strongly suggests that reduced thioredoxin inhibits peroxidation of lipids through its interaction with the iron via the sulfhydryls at the active site. This action of thioredoxin is unique since it is documented in the literature that thiol compounds (i.e., cysteine, glutathione, DTT) can cause and promote lipid peroxidation (Rowley, D:A. and Halliwell, B. [1982] FEBS Lett. 138:33-66; Bucher et al. [1982] in "Oxy Radicals and Their Scavenger Systems" Vol. 1, pp. 370-363 [Cohen, G. and Greenwald, R.A., ed.]; Tien et al. [1982] Biochem Biophys. Res. Comm. 107:279-285).

It is noteworthy that reduced thioredoxin was more effective (approximately two-fold) than desferrioxamine. Since desferrioxamine inhibits by chelation or iron, reduced thioredoxin must have some anti-oxidant action different than, or in addition to, iron chelation.

Example 8

Lipid peroxidation studies were carried out as described in Example 7 using a dithiol peptide with the sequence of E . coli thioredoxin active site. The peptide, $T_{31-36}$, Trp-Cys-Gly-Pro-Cys-Lys, was synthesized by conventional solid phase procedures such as that described by Merrifield, J.Am. Chem Soc. (1964) 85:2149. The ability of the $T_{31-36}$ peptide to inhibit lipid peroxidation was similar to the efficacy of the intact protein. The oxidized peptide inhibited MDA formation proportional to peptide concentration (i.e., as observed for both oxidized thioredoxin and desferrioxamine. The reduced peptide completely inhibited MDA formation at approximately 0.05 mM (i.e., as observed for reduced thioredoxin).

Also included within the scope of the present invention are the pharmaceutically acceptable salts of thioredoxin compounds, such as sodium or potassium or those made with strong organic bases such as guanidine. In addition, the counter ions of these cations as well as of lysine residues in thioredoxin compounds such as the hydrochloride, hydrobromide sulfate, phosphate, maleate, acetate, citrate, benzoate, succinate, malate, ascorbate and the like, may be included in the preparation.

Also included within the scope of this invention are peptides containing the dithiol sequence (Trp)-Cys-X-Y-Cys-Lys with amino and/or carboxyl blocking groups, particularly those groups that render the charge neutral. Examples of amino blocking groups include acyl 1-18C, e.g., acetyl and t-butoxycarbonyl; carboxy blocking groups include lower alkyl esters, e.g., methyl and ethyl, and amide groups.

The acyls can be prepared by use of standard acylating conditions well know to those skilled in the art. Acids which can be used in the acylation include (a) saturated or unsaturated, straight or branched chain aliphatic carboxylic acids, for example, acetic, propionic, butyric, isobutyric, tert-butylacetic, valeric, isovaleric, caproic, caprylic, decanoic, dodecanoic, lauric, tridecanoic, myristic, pentadecanoic, palmitic, margaric, stearic, acrylic, crotonic, undecylenic, oleic, hexynoic, heptynoic, octynoic acids, and the like; (b) saturated or unsaturated, alicyclic carboxylic acids, for example, cyclobutanecarboxylic acid, cyclopentanecarboxylic acid, cyclopentenecarboxylic acid, methylcyclopentenecarboxylic acid, cyclohexanecarboxylic acid, dimethylcyclohexanecarboxylic acid, dipropylcyclohexanecarboxylic acid, and the like; (c) saturated or unsaturated, alicyclic aliphatic carboxylic acids, for example, cyclopentaneacetic acid, cyclopentanepropionic acid, cyclohexaneacetic acid, cyclohexanebutyric acid, methylcyclohexaneacetic acid, and the like; (d) aromatic carboxylic acids, for example, benzoic acid, toluic acid, naphthoic acid, ethylbenzoic acid, isobutylbenzoic acid, methylbutylbenzoic acid, and the like; and (e) aromatic-aliphatic carboxylic acids, for example, phenylacetic acid, phenylpropionic acid, phenylvaleric acid, cinnamic acid, phenypropiolic acid and naphthylacetic acid, and the like.

**Claims**

1. A dithiol peptide selected from thioredoxin and derivatives and analogues thereof, for use in therapy.

2. A dithiol peptide according to claim 1, which is thioredoxin from E. coli or mammalian thioredoxin.

3. A dithiol peptide according to claim 1, which is the fragment containing residues 1 to 37 (as produced by cyanogen bromide cleavage) or E. coli thioredoxin, or the fragment containing residues 19 to 36 (as produced by trypsin digestion of residues 1 to 37 produced by cyanogen bromide cleavage) of E. coli thioredoxin.

4. A dithiol peptide according to any preceding claim, which comprises the redox-active peptide sequence Cus-X-Y-Cys, wherein X and Y are independently selected from the 20 naturally-occurring amino-acids.

5. A diothiol peptide according to claim 4, wherein the redox-active peptide sequence is Cys-X-Y-Cys-Lys, Trp-Cys-X-Y-Cys-Lys, A-Cys-X-Y-Cys-Lys-B, A-Cys-X-Y-Cys-B or A-Trp-Cys-X-Y-Cys-Lys-B, wherein A is an amino-blocking group, B is a carboxyl-blocking group, and X and Y are as defined in claim 4.

6. A dithiol peptide selected from derivatives and analogues of thioredoxin, which comprises the redox-active peptide sequence Trp-Cys-X-Y-Cys-Lys or A-Trp-Cys-X-Y-Cys-Lys-B, wherein A and B are as defined in claim 5 and X and Y are as defined in claim 4.

7. A dithiol peptide according to any of claims 4 to 6, wherein X is Gly and Y is Pro.

8. Use of a dithiol peptide as defined in any preceding claim, for the manufacture of a medicament for inhibiting metal-catalysed oxidation in a biological system.

9. Use of a dithiol peptide as defined in any of claims 1 to 7, for the manufacture of a medicament for the treatment of ischemia or an inflammatory condition.

10. A non-therapeutic process for inhibiting metal-catalysed oxidation in a biological system, which comprises applying a dithiol peptide according to any of claims 1 to 7.

11. A process according to claim 10, wherein the system comprises lipids in solution or in membranes, food, drugs, or cosmetics.